(19) Europäisches Patentamt
European Patent Office
Office européen des brevets

(11) Publication number: 0 201 751
A2

(12) # EUROPEAN PATENT APPLICATION

(21) Application number: 86105340.3

(22) Date of filing: 17.04.86

(51) Int. Cl.⁴: C 09 B 11/08
G 01 N 33/535, C 09 B 11/10

(30) Priority: 08.05.85 US 732461
08.05.85 US 732462

(43) Date of publication of application:
20.11.86 Bulletin 86/47

(84) Designated Contracting States:
BE DE FR IT

(71) Applicant: ABBOTT LABORATORIES
14th Street and Sheridan Road North St
North Chicago Illinois 60064(US)

(72) Inventor: Fino, James Robert
231 Southwick Court
Vernon Hills Illinois 60061(US)

(72) Inventor: Shipchandler, Mohammed Tyebji
640 Burdick Street
Libertyville Illinois 60048(US)

(74) Representative: Modiano, Guido et al,
MODIANO, JOSIF, PISANTY & STAUB Modiano &
Associati Via Meravigli, 16
I-20123 Milan(IT)

(54) **4'-aminomethyfluorescein derivatives for use in fluorescence polarization immunoassys.**

(57) The present invention is directed to an improved method for the preparation of 4'-aminomethylfluorescein derivatives for use in fluorescence polarization immunoassays. The invention is also directed to a novel class of 4'-N-alkylamino-methylfluorescein derivatives possessing utility as reagents in such assays.

EP 0 201 751 A2

Croydon Printing Company Ltd.

# 4' - AMINOMETHYLFLUORESCEIN DERIVATIVES FOR USE IN FLUORESCENCE POLARIZATION IMMUNOASSAYS

## Background of the Invention

### Technical Field

The present invention relates in a first aspect, to an improved method for the preparation of 4'-aminomethylfluorescein derivatives and, in a second aspect, to a novel class of 4'-N-alkylaminomethylfluorescein derivatives, both of which may be employed as reagents in fluorescence polarization immunoassays.

### Background Art

Competitive binding immunoassays provide a means for determining the concentration of ligands in biological fluids such as serum, plasma, spinal and amniotic fluids, and urine.

The ligand to be measured competes with a labeled reagent, or "ligand-analog" or "tracer", for a limited number of receptor binding sites on antibodies specific to the ligand and ligand-analog. For purposes of this disclosure, the term "ligand-analog" refers to a compound possessing one or more determinant or epitopic sites capable of competing with the ligand for the binding sites of a receptor, whether in conjugation with a fluorescein derivative to form a tracer or in its unconjugated form. The concentration of ligand in the sample determines the amount of ligand analog which binds to the antibody: the amount of ligand analog that will bind is inversely proportional to the concentration of ligand in the sample, because the ligand and the tracer each bind to the antibody in proportion to their respective concentrations.

Fluorescence polarization provides a quantitative means for measuring the amount of tracer-antibody conjugate produced in a competitive binding immunoassay. Fluorescence polarization

techniques are based on the principle that a fluorescent labeled compound, when excited by plane polarized light, will emit fluorescence having a degree of polarization inversely related to its rate of rotation. Accordingly, when a tracer-antibody conjugate having a fluorescent label is excited with plane polarized light, the emitted light remains highly polarized because the fluorophore is constrained from rotating between the time that light is adsorbed and emitted. In contrast, when an unbound tracer is excited by plane polarized light, its rotation is much faster than that of the corresponding tracer-antibody conjugate. As a result, the light emitted from the unbound tracer molecules is depolarized.

The use of 4'-aminomethylfluorescein derivatives as tracers in fluorescence polarization immunoassays is known in the art, having been disclosed in U.S. Patent No. 4,510,251 to Kirkemo and Shipchandler. The method disclosed therein for the preparation of 4'-aminomethylfluorescein is not without drawbacks, however, as it has subsequently been learned by applicants that alkylation occurs as an unavoidable side reaction, thereby diminishing the yield of 4'-aminomethylfluorescein. The present invention provides a method whereby 4'-aminomethylfluorescein can be prepared without concomitant alkylation and thus with greatly improved yield.

In addition, a novel class of 4'-aminomethylfluorescein derivatives, viz., 4'-N-alkylaminomethylfluorescein compounds, has been discovered as an aspect of this invention. These unique and unexpected fluorescein derivatives possess greater sensitivity, and hence utility, than 4'-aminomethylfluorescein in certain specific instances, as will be explained infra.

Summary of the Invention

A first aspect of the invention relates to the discovery of a class of 4'-N-alkylaminomethylfluorescein derivatives having the structural formula:

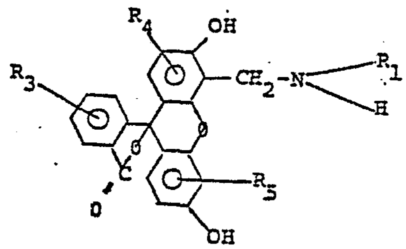

and acid addition salts thereof, wherein $R_1$ is $C_1$ to $C_8$ alkyl; $R_3$ is hydrogen, alkyl, halo, amino or carboxyl; $R_4$ is hydrogen, alkyl, halo, amino or carboxyl, and $R_5$ is hydrogen, alkyl, halo, amino or carboxyl.

These 4'-N-alkylaminomethylfluorescein derivatives possess particularly useful properties for certain fluorescence polarization immunoassays, wherein they are utilized as fluorescent tracers having the formula:

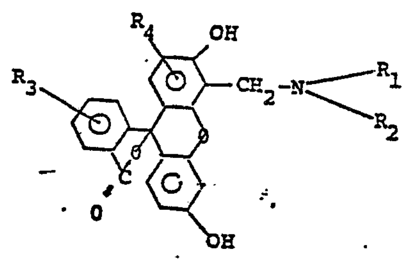

and the acid addition salts thereof, wherein $R_1$, $R_3$, $R_4$, and $R_5$ are as defined above, and $R_2$ is a ligand-analog having at least one common epitope with a ligand so as to be specifically recognizable by a common

antibody. The use of the derivatives in fluorescence polarization immunoassays represents a second aspect of the invention.

A third aspect of the invention relates to a method for the preparation of 4'-N-alkylaminomethylfluorescein compounds and their acid addition salts.

A fourth aspect of the invention relates to a new and improved method for the preparation of 4'-aminomethylfluorescein compounds having the structural formula:

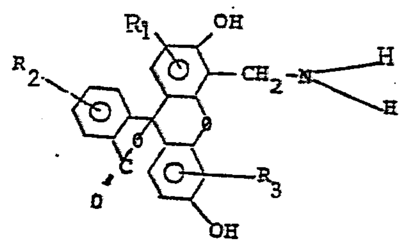

and acid addition salts thereof, wherein $R_1$, $R_2$ and $R_3$ each may be hydrogen, alkyl, halo, amino or carboxyl.

The novel process whereby these compounds may be prepared comprises reacting an acetamidomethylfluorescein or 4'-haloacetamidomethylfluorescein in the presence of a nonhydroxylic ether and a strong acid and recovering the 4'-aminomethylfluorescein thus produced.

Further objects and attendant advantages of the invention will be best understood from a reading of the following detailed description, taken together with the figures and examples.

## Detailed Description of the Invention

The present invention involves the use of fluorescein and derivatives of fluorescein and, more specifically, utilization of their ability to emit fluorescence. Fluorescein exists in either of two tautomeric forms depending on the acid concentration

(pH) of the environment. In the open (acid) form, there are a number of conjugated double bonds which make that form of fluorescein (and compounds containing a fluorescein moiety) capable of absorbing blue light and emitting green fluorescence after an excited state lifetime of about four nanoseconds. As used herein, the term "fluorescein", either as an individual compound or as a component of a larger compound, is meant to include both the open and closed forms, if they exist for a particular molecule, except in the context of fluorescence. An open form is necessary for the fluorescence to occur.

A tracer in solution which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes rotation corresponding to the polarization observed. When a ligand competes with a tracer for antibody sites, then the observed polarization of fluorescence of the resulting mixture of free tracer and tracer-antibody complex assumes a value intermediate between that of the tracer and that of the tracer-antibody complex. If a sample contains a high concentration of the ligand, the observed polarization value is closer to that of the free ligand, i.e., low. If the test sample contains a low concentration of the ligand, the polarization value is closer to that of the bound ligand, i.e., high. By sequentially exciting the reaction mixture of an immunoassay with vertically and then horizontally polarized light and analyzing only the vertically polarized component of the emitted light, the

polarization of fluorescence in the reaction mixture may be accurately determined. The precise relationship between polarization and concentration of the ligand to be determined is established by measuring the polarization values of calibrators with known concentrations. The concentration of the ligand can be interpolated from a standard curve prepared in this manner.

The 4'-N-alkylaminomethylfluorescein tracers of the present invention generally exist in an equilibrium between their protonated and ionized states, and in the ionized state are effective in fluorescence polarization immunoassays. For convenience, the tracers are structurally represented herein in their protonated form. In their ionized state, the tracers exist in the form of biologically acceptable acid addition salts. As used herein, the term "biologically acceptable salts" refers to salts such as sodium, potassium, and ammonium which will enable the tracers to exist in their ionized state when employed in fluorescence polarization immunoassays. Generally, the tracers of the present invention exist in solution as salts, the specific salt resulting from the buffer employed. For example, in the presence of a sodium phosphate buffer, the tracers will generally exist in their ionized state as sodium salts.

Various 4'-aminomethylfluorescein derivatives useful as tracers can be prepared in accordance with the improved method of the present invention. Essentially, for all but the 4'-N-alkylaminomethylfluorescein derivatives, fluorescein is treated with a 4'-haloacetamidomethanol in the presence of acid to yield an acetamidomethyl or 4'-haloacetamide derivative, which is then hydrolyzed in the presence of acid and a nonhydroxylic ether to yield an acid salt of a 4'-aminomethylfluorescein. No undesired alkylated side

products are produced by this method. The 4'-aminomethylfluorescein so produced is treated with an activated ester of the ligand-analog of choice in the presence of a suitable solvent and a suitable base sufficient to neutralize the acid salt to yield 4'-aminomethylfluorescein tracer. The nonhydroxylic ether that is used will preferably have a boiling point in the range of 80-200°C. Examples of preferred ethers include diethylene glycol dimethyl ether and ethylene glycol dimethyl ether.

The 4'-N-alkylaminomethylfluorescein derivatives of the present invention are, however, prepared in accordance with the general procedures set forth in Examples III, IV and V. For these derivatives, fluorescein is treated with a 4'-haloacetamidomethanol in the presence of acid to yield a 4'-haloacetamide derivative, which is then hydrolyzed in the presence of acid and a $C_1$-$C_8$ alcohol. This yields an acid salt of 4'-N-($C_1$-$C_8$ alkyl)aminomethylfluorescein derivative corresponding to the particular $C_1$-$C_8$ alcohol used in hydrolysis. The alkylation of the amino group upon removal of the chloroacetyl group during hydrolysis was an unexpected result. There are no instances known to the applicants wherein an amino group is alkylated by an alcohol under acidic conditions. The 4'-N-alkylaminomethylfluorescein so produced is treated with an activated ester of the ligand-analog of choice in the presence of a suitable solvent and a suitable base sufficient to neutralize the acid salt.

The temperature at which the reaction for preparing the tracer proceeds is not critical, so long as it is sufficient to initiate and maintain the reaction without causing unwanted degredation or side reactions. Generally, for convenience and economy, room temperature will suffice.

The pH must be sufficient to allow tracers prepared according to the present invention to exist in their ionized state. The pH may range from about 3 to 12, more usually in the range of from 5 to 10, most preferably from about 6 to 8. Various buffers may be used to achieve and maintain the pH during the fluorescence polarization immunoassay procedure. Representative buffers include borate, phosphate, carbonate, tris, barbital, and the like. The particular buffer employed is not critical as to the 4'-aminomethylfluorescein derivatives, but in an individual assay a specific buffer may be preferred in view of the antibody and the ligand to be determined. As stated earlier, the cation portion of the buffer will generally determine the cation portion of the tracer salt in solution.

The concentration of ligand which may be assayed will generally vary from about $10^{-2}$ to $10^{-13}$ M, more usually from about $10^{-4}$ to $10^{-10}$ M. Higher concentrations of ligand may be assayed upon dilution of the original sample. In addition to the concentration range of ligand of interest, considerations such as whether the assay is qualitative, semiquantitative, or quantitative, the equipment employed, and the characteristics of the tracer and antibody will normally determine the concentration of the tracer and antibody to be employed, as will be apparent to those skilled in the art.

A sample containing the ligand to be determined is intermixed with a biologically acceptable salt of a tracer and an antibody specific for the ligand and tracer. The ligand present in the sample and the tracer compete for limited antibody sites, resulting in the formation of ligand-antibody and tracer-antibody complexes. Because the concentration of tracer and

antibody is maintained constant, the ratio of ligand-antibody complex to tracer-antibody complex formed is directly proportional to the amount of ligand present in the sample. Therefore, upon exciting the mixture with fluorescent light and measuring the polarization of the fluorescence emitted by a tracer and a tracer-antibody complex, one is able to quantitatively determine the amount of ligand in the sample.

Examples of ligands determinable by 4'-aminomethylfluorescein tracers prepared according to the present invention and by the novel 4-N-alkylaminomethylfluorescein tracers of a second aspect of the invention include steroids such as estriol, estradiol, cortisol, testosterone and progesterone; vitamins; various kinds of drugs and their metabolites; and other ligands enumerated in U.S. Patent No. 4,510,251.

Immunogens used in antibody production are often produced by attaching the carboxylic acid "arm" of a hapten to the epsilon amino group of the lysines of a large molecular weight protein. This attachment occurs through the formation of an amide bond, or "bridge". It has been observed that certain species of polyclonal antibodies are attracted to this bridge rather than to the hapten itself. A tracer having this particular bridge may become so tightly bound to antibody that it cannot be displaced effectively by the hapten. This situation has been described by J.E.T. Corrie and W.M. Hunter in Methods of Enzymology, 73, 79 (1981) as the "bridge binding phenomenon". This problem can be circumvented by using the tracers of the present invention, wherein the amide bond has been altered by the substitution of a $C_1$-$C_8$ alkyl group for a hydrogen on the nitrogen.

Another problem sometimes encountered in fluorescence polarization immunoassays involves quenching of the fluorescent signal of the tracer by the hapten, resulting in decreased sensitivity. While applicants do not wish to be bound to the following theory, it is believed that the proximity of the fluorophore to the hapten creates a "folding over" effect resulting in nonradiative energy transfer, and that the placement of a bulky group on the amide bridge will lessen this folding over and minimize energy transfer. Thus, the 4'-N-alkylaminomethylfluorescein tracers are recommended over their nonalkylated counterparts in situations in which either the bridge binding phenomenon or the folding over effect is encountered.

The following illustrative, nonlimiting examples will serve to further demonstrate to those skilled in the art the manner in which specific tracers may be prepared in accordance with the invention.

Example I

This example illustrates the preparation of 4'-chloroacetamidomethylfluorescein, which serves as an intermediate in the preparation of 4'-aminomethylfluorescein.

To 10.0 g (30.1 mmole) of fluorescein dissolved in 100 ml of concentrated sulfuric acid was added 3.7 g (30.1 mmole) of N-hydroxymethylchloroacetamide as a solid, along with 100 ml of additional sulfuric acid. The reaction was stirred for 20 hours under an argon atmosphere protected from light and then poured over 1 liter of ice. The yellow precipitate was washed several times with ice-cold water and then dried under reduced pressure. The product was purified using column chromatography (7 x 50 cm column, 700 g silica gel). Elution was with 5% methanol in methylene chloride,

followed by 10% methanol in methylene chloride. Fractions wee checked by thin-layer chromatography (silica gel 10% methanol in methylene chloride) after which the appropriate fractions were combined and the solvent was removed under reduced pressure. The yield was 4.9 g (37%) of chloroacetamidomethylfluorescein as an orange solid.

Example II

This example illustrates the preparation of 4'-aminomethylfluorescein.

Chloroacetamidomethylfluorescein (25.0 g) prepared according to Example 1, was refluxed in diethylene glycol dimethyl ether (110 ml) and concentrated hydrochloric acid (30 ml) for 20 hours under a nitrogen atmosphere. The solvent was removed under reduced pressure and the residue was purified using a Waters Prep LC/System 500 A liquid chromatograph equipped with two Prep PAK C18 columns. Elution at 100m/minute was with water:methanol:acetic acid (50:50:0.4). The residue was dissolved in methanol (40 ml) and four injections were made into the preparative system. Fractions were checked by thin-layer chromatography (Whatman C18 plates, 40:60:0.4, 500 mM ammonium acetate:methanol:acetic acid), after which the appropriate fractions were combined. The solvent was removed under reduced pressure and the residual acetic acid was removed by coevaporation with methanol:toluene (1:1.3 x 100 ml). The yield was 6.8 g (31%) of 4'-aminomethylfluorescein hydrochloride as a dark orange solid.

Example III

This example illustrates the preparation of 4'-N-methylaminomethylfluorescein.

A solution of 4'-chloroacetamidomethylfluorescein (2.4 g), prepared according to Example I, in methanol (40 ml) and concentrated hydrochloric acid (3 ml) was refluxed for 20 hours under argon atmosphere. The solvent was removed under reduced pressure and the residue was purified using a Waters Prep LC/System 500A liquid chromatograph equipped with two one-inch columns packed with reverse-phase medium (Whatman Partisil-40, ODS-3). Elution was with water:methanol:acetic acid (45:55:0.5) at 50 ml/minute. Fractions were checked by thin-layer chromatography (Whatman C18 plates, same solvent system), after which the appropriate fractions were combined and the solvent was removed under reduced pressure. The residue was crystallized from water:methanol (1:1, 15 ml) to yield 0.32 g (16%) of 4'-N-methylaminomethylfluorescein hydrochloride as an orange solid.

<u>Example IV</u>

This example illustrates the preparation of 4'-N-ethylaminofluorescein.

A solution of 4'-chloroacetamidomethylfluorescein (3.0 g), prepared according to Example I, in 95% ethanol (50 ml) and concentrated hydrochloric acid (10 ml) was refluxed for 20 hours under an argon atmosphere. The solvent was removed under reduced pressure and the residue was purified by column chromatography (7 x 50 cm column, 300 g silica gel) eluting with methanol:methylene chloride (30:70). Fractions were checked by thin-layer chromatography (silica gel, same system), after which the appropriate fractions were combined and the solvent was removed under reduced pressure. The yield was 1.5 g (55%) of 4'-N-ethylaminomethylfluorescein hydrochloride as an orange solid.

Example V

This example illustrates the preparation of 4'-N-n-butylaminomethylfluorescein.

A solution of 4'-chloroacetamidomethylfluorescein (19.0 g), prepared according to Example I, in n-butanol (160 ml) and concentrated hydrochloric acid (30 ml) was refluxed for 6 hours under a nitrogen atmosphere. The reaction was stopped so as to produce both 4'-aminomethylfluorescein and 4'-N-n-butylaminomethyl fluorescein in an approximately 60:40 mixture by thin-layer chromatography (Whatman C18 plates, water:methanol:acetic acid:40:60:0.5). The solvent was removed under reduced pressure, yielding a dark red, gel-like residue. This residue was purified using a Waters Prep LC/System 500 A liquid chromatograph equipped with two Prep PAK-500/C18 columns. Elution was with water:methanol:acetic acid (45:55:0.5) at 100 ml/minute. The residue was dissolved in methanol (40 ml) and two injections were made, one approximately 30 ml and the other approximately 20 ml. Fractions were checked by thin-layer chromatography (same system), after which the appropriate fractions were combined and the solvent removed under reduced pressure, yielding 6.0 g (38%) of 4'-aminomethylfluorescein hydrochloride and 5.55 g (30.9%) 4'-N-n-butylaminomethylfluorescein hydrochloride.

Example VI

This example illustrates the preparation of an estriol tracer utilizing the 4'-N-ethylaminomethylfluorescein prepared in Example IV.

To a solution of 0.100 g of estriol-3-carboxymethylether and 0.050 g of N-hydroxysuccinimide in dry N,N-dimethylformamide was added 0.060 g of N,N'-dicyclohexylcarbodiimide. The solution was stirred at room temperature under an inert atmosphere

overnight. This solution was added to 0.123 g of 4'-ethylaminomethylfluorescein which had been dissolved in dry N,N-dimethylformamide and 0.060 ml of triethylamine. The reaction was again stirred at room temperature under an inert atmosphere overnight, after which the solvent was removed under reduced pressure. The residue was purified by preparative thin-layer chromatography, first with reverse-phase medium (methanol:water:acetic acid), then with silica gel (methanol:methylene chloride:acetic acid, 10:90:0.4), to yield estriol-3-carboxymethylether-4'-N-ethylaminomethylfluorescein tracer. This tracer was successfully utilized in a fluorescence polarization immunoassay for total estriol.

It will be understood that various changes, modifications and equivalents can be made in the details of procedure, formulation and use without departing from the spirit and scope of the invention.

CLAIMS:

1. A compound having the formula:

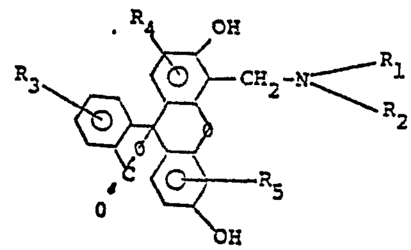

and the acid additional salts thereof, wherein $R_1$ is $C_1$ to $C_8$ alkyl; $R_2$ is a ligand-analog having at least one common epitope with a ligand so as to be specifically recognizable by a common antibody; $R_3$ is hydrogen, alkyl, halo, amino or carboxyl; $R_4$ is hydrogen, alkyl, halo, amino or carboxy; and $R_5$ is hydrogen, alkyl, halo, amine or carboxyl.

2. A compound as defined in Claim 1 wherein $R_1$ is a methyl group.

3. A compound as defined in Claim 1 wherein $R_1$ is an ethyl group.

4. A compound as defined in Claim 1 wherein $R_1$ is an n-butyl group.

5. A process for the preparation of a compound having the formula:

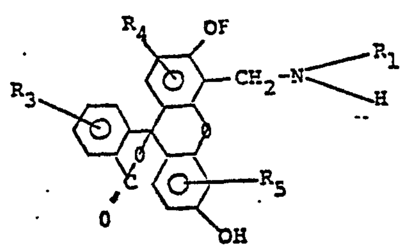

and the acid addition salts thereof, wherein $R_1$ is $C_1$ to $C_8$ alkyl; $R_3$ is hydrogen, alkyl, halo, amino or carboxy; $R_4$ is hydrogen, alkyl, halo, amino or carboxyl; and $R_5$ is hydrogen, alkyl, halo, amino or carboxy; comprising reacting a haloacetamidomethylfluorescein the the presence of an alcohol and a strong acid and recovering the 4'-N-alkylaminomethylfluorescein compound thus produced.

6. A process as defined in Claim 5 wherein the alcohol is methanol.

7. A process as defined in Claim 5 wherein the alcohol is ethanol.

8. A process as defined in Claim 5 wherein the alcohol is n-butanol.

9. In a method of detecting a ligand by fluorescence polarization using a fluorescent tracer, the improvement comprising using as a tracer a compound as defined in Claim 1.

10. A process for the preparation of 4'-aminomethylfluorescein compounds, and the acid addition salts thereof, comprising reacting an acetamidomethylfluorescein or 4'-haloacetamidomethylfluorescein in the presence of a nonhydroxylic ether and a strong acid and recovering the 4'-aminomethylfluorescein compound thus produced.